**Europäisches Patentamt**

**European Patent Office**

**Office européen des brevets**

(19)

(11) Veröffentlichungsnummer: **0 310 923 B1**

# EUROPÄISCHE PATENTSCHRIFT

(12)

(45) Veröffentlichungstag der Patentschrift: 07.08.91 Patentblatt 91/32

(51) Int. Cl.$^5$: **A61F 13/20**

(21) Anmeldenummer: **88115959.4**

(22) Anmeldetag: **28.09.88**

(54) **Verfahren zur Herstellung von Gegenständen mit Saugfähigkeit unter Verwendung von Fasermaterial mit Anteilen von wenigstens an ihrer Oberfläche schmelzbaren Fasern und Anlage zur Durchführung des Verfahrens.**

(30) Priorität: **09.10.87 DE 3734184**

(43) Veröffentlichungstag der Anmeldung: **12.04.89 Patentblatt 89/15**

(45) Bekanntmachung des Hinweises auf die Patenterteilung: **07.08.91 Patentblatt 91/32**

(84) Benannte Vertragsstaaten: **AT BE DE FR LU NL SE**

(56) Entgegenhaltungen:
DE-A- 1 566 333
DE-A- 2 634 572
DE-A- 2 846 593
FR-A- 2 467 165

(73) Patentinhaber: **W. PELZ GMBH & CO.**
**Dr.-Hermann-Lindrath-Strasse**
**W-2362 Wahlstedt/Holstein (DE)**

(72) Erfinder: **Pelz, Udo W.**
**Parkstr. 20a**
**W 2360 Bad Segeberg (DE)**

(74) Vertreter: **Siemons, Norbert, Dr.-Ing. et al**
**Neuer Wall 41**
**W-2000 Hamburg 36 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 310 923 B1

**Beschreibung**

Die Erfindung betrifft ein Verfahren zur Herstellung von Gegenständen mit Saugfähigkeit unter Verwendung von Fasermaterial, wie Baumwolle, Viskose, mit Anteilen von wenigstens an ihrer Oberfläche schmelzbaren Fasern, bei dem Formstücke entsprechend dem Gegenstand aus einem Fasermaterialband aus dem Fasermaterial ausgestanzt oder geschnitten werden und ein Verschnitt als Abfall anfällt, ferner diese Abfallmaterialien in eine Fertigungsstrecke zur Herstellung eines Fasermaterialbandes zurückgeführt werden und im Verlauf dieses Verfahrens Wärme aufgebracht wird, durch welche die wenigstens an ihrer Oberfläche schmelzbaren Fasern verbunden werden.

Ferner bezieht sich die Erfindung auf eine Anlage zur Durchführung des Verfahrens mit einer Fertigungsstrecke für die Herstellung eines Wattebandes mit einer Erwärmungsvorrichtung zum Zusammenschluß des Wattebandes durch Schmelz- und Verklebungsverbindungen zwischen Fasern an Kreuzpunkten und einer Stanzvorrichtung zum Ausstanzen von Wattetupfern sowie einer Transportstrecke zur Rückführung von Abfallmaterialien in der Fertigungsstrecke.

Ein Watteband zur Herstellung von solchen Tupfern besteht beispielsweise aus Baumwolle oder Viskose und Schmelzfasern. Beispielsweise werden dabei 80% Baumwolle oder 80% Viskose zum Beispiel 1,5 bis 3,5 dtex, Stapellänge etwa 40 mm und 20% Schmelzfaser, zum Beispiel Type Danaklon ES, zum Beispiel 1,7 bis 2,2 dtex, Stapellänge etwa 40 mm zusammengetragen. Hierin besteht eine vorteilhafte Ausführung, die zugrunde gelegt werden kann. Es können aber auch entsprechende andere Materialien, auch mit Abweichungen der Anteile verwendet werden. Der Zusammenschluß wird durch eine Heißluftdurchströmung mit einer Temperatur in der Größenordnung von 140°C herbeigeführt. In den bekannten Verfahren und Anlagen werden nach dem Zusammenschluß die Wattetupfer als runde Wattescheiben mit einem Durchmesser von beispielsweise 60 mm ausgestanzt. Hierbei können in einem Watteband bei entsprechender Bemessung bekanntlich drei Scheiben nebeneinander ausgestanzt werden. Schon bei Ausstanzung runder Scheiben ergibt sich ein erheblicher Abfallverlust aus dem mit Planrändern hergestellten Watteband. Bei der Ausstanzung dreier Scheiben nebeneinander ergibt sich je nach Verfahrensweise ein Watteabfall von etwa 35 bis 45% des Wattebandes.

Vorstehende Gesichtspunkte hinsichtlich der Materialien, der Umrißform der Wattetupfer und des Watteabfalls bezieht die Erfindung ein.

Der Ausdruck Watte ist dabei nicht auf die angegebenen Materialien beschränkt, sondern es wird lediglich einbezogen, daß die den Saugkörper bildenden Fasern mit sogenannten Schmelzfasern durchsetzt werden.

Bei den meisten bekannten Verfahren erfolgt der Zusammenschluß durch Wärmeaufbringung vor dem Ausstanzen. Der anfallende Watteabfall, der einen erheblichen Anteil des zugeführten Materials ausmacht, weist daher bereits durch Verschmelzung oder Verklebung zusammengeschlossene Fasern auf.

Aus der DE-A-2634572 — sie liegt dem Oberbegriff des Verfahrens-u, Vorrichtungs-anspruchs zugrunde — ist Verarbeitung von Abfallprodukten bekannt, die zu Teilchen zerkleinert werden, diese Teilchen nach Gewicht getrennt werden und dann die schweren Teilchen für die Fasergewebeherstellung verwendet und die zerkleinerten Teilchen mittels Unterdruck an ein Förderband angesaugt werden, wobei dann eine Kalandrierung des Fasergewebes beim Pressen und Erwärmen erfolgt. Hierbei erfolgt die Verarbeitung an beheizten Kalandern, wobei die thermoplastischen Fasern verschmolzen und dann auch die als Abfallmaterialien zugeführten Teile nun schon aufgeschmolzene Fasern besitzen. Dadurch ist bei den Erzeugnissen, die damit ausgestattet werden, eine geringere Qualität, insbesondere im Zusammenhang mit einer sogenannten Knötchenbildung vorhanden.

Wenn es bekannt ist, Abfallmaterialien in den bekannten Verfahren zur Herstellung des Wattebandes zurückzuführen, ergibt sich eben der Nachteil von Bestandteilen aus Fasern, die nicht mehr locker zusammengeschichtet sind, sondern bereits verschmolzen oder verklebt sind, so daß das Enderzeugnis der Wattetupfer Materialabschnitte mit unterschiedlichen Eigenschaften aufweist, und die durch die Abfallmaterialien gebildeten Materialabschnitte verfestigte Stellen bilden, die nicht in gleicher Weise in den Tupfer eingebunden sind, wie die Baumwoll- bzw. Viskosefasern und Schmelzfasern in einem noch ungebundenen Zustand. Nachteile entstehen auch dadurch, daß die Fasern an den Schmelz- und Verklebungspunkten reißen. Die Fasern werden in der Faserlänge kürzer. Wenn aber die thermoverfestigten Fasern aufgelöst werden sollen, wird dadurch ein erheblicher Energieaufwand benötigt. Überhaupt ist bei den bekannten Ausführungen zu berücksichtigen, daß die aufgebrachte Energie auf das Watteband insgesamt, d.h. einschließlich der Abfallanteile, erheblich ist und daß ein entsprechender Energieanteil nicht nur nutzlos verbraucht wird, sondern auch letzten Endes bei einer Rückführung zu einem immer schlechteren Ausgangsmaterial beiträgt. Wenn ein Kardierprozeß bei der Wattebandherstellung eingesetzt ist, ergeben sich infolge der nicht aufgelösten Schmelz- und Verklebungspunkte stärkere Beanspruchungen der Kadiergarnituren, so daß deren Lebensdauer verkürzt wird.

Der Erfindung liegt die Aufgabe zugrunde, ein Verfahren und eine Anlage zur Herstellung von Wattetupfern der eingangs angegebenen Art zu schaffen, welche die Nachteile der bekannten Ausführungen beseitigt und insofern energiesparend wirkt, zur Verbesserung der Qualität der Wattetupfer auch bei Rückführung von Abfallmaterialien gegenüber bekannten Ausführungen beiträgt und die Aggregate der Fertigungsstrecke schont.

Diese Aufgabe wird durch das Verfahren dadurch gelöst, daß als Gegenstände mit Saugfähigkeit Wattetupfer aus einem Watteband aus Fasermaterial mit Anteilen von wenigstens an ihrer Oberfläche schmelzbaren Fasern ausgestanzt und erst danach die bereits ausgestanzten Wattebandabschnitte, die jeweils einen Tupfer bilden, einer die Tupfer verfestigenden Wärmeaufbringung ausgesetzt werden, und daß das Abfallmaterial nach der Ausstanzung und vor der Wärmeaufbringung zurückgeführt wird.

Hierdurch wird die Umrißform des Wattetupfers, insbesondere die kreisförmige Scheibe in unverbundenem Zustand ausgestanzt, ohne daß Verbindungsenergie auf das gesamte Watteband aufgebracht wird. Wenn nachträglich nur die ausgestanzten Wattescheiben thermoverfestigt werden, wird in Proportionen zur Menge der Abfallmaterialien eine Energieeinsparung in der Größenordnung von 35 bis 45% erreicht, und zwar hinsichtlich der zum Zusammenschluß der verschiedenen Faseranteile aufgebrachten Energie.

Es ist zwar aus der DE-A-2846593 bekannt, bei Herstellung von Wattetupfer die Wärmeaufbringung nach Beschneiden der Wattebahn vorzunehmen ; jedoch erfolgt diese Reihenfolge der Verfahrensschritte im Hinblick auf eine verbesserte Qualität des Wattetupfers als Absorptions-Körper. Dem Dokument ist nichts über eine verbesserte Aufbereitung des Abfallmaterials zu entnehmen.

Der weitere Vorteil besteht darin, daß das ausgestanzte Abfallmaterial problemlos wieder zugeführt werden kann. Insofern liegt ein Merkmal des Verfahrens auch darin, daß die durch das Ausstanzen anfallenden Abfallmaterialien in die Fertigungsstrecke zur Herstellung des Wattebandes zurückgeführt werden.

Wenn oben bestimmte Umrißformen angeführt sind, werden beispielsweise mehreckige, wie sechsoder achteckige oder andere Umrißformen mit gekrümmten Randlinien, wie zum Beispiel Blütenund Blattformen einbezogen. Bevorzugt werden aber auch im erfindungsgemäßen Verfahren Tupfer mit kreisförmigen Umfang verschiedenen Durchmessers, so daß dann das obengenannte Verhältnis entsteht.

Wenn eine Rückführung der Abfallmaterialien vorgesehen ist und die oben angeführten Nachteile der in bekannten Ausführungen verklebten oder verschmolzenen Abfallmaterialien berücksichtigt werden, dann wird eine weitere Energieeinsparung und Vereinfachung der Anlage dadurch erreicht, daß die Abfallmaterialien vor der Einführung in die Fertigungsstrecke nur aufgelockert werden. Einer Auflockerung der unverbundenen Faserbestandteile führt diese nicht nur in ihre originäre Form zurück, sondern vermeidet überhaupt Energie, die sonst zum Aufreissen der Abfallmaterialien aufgebracht werden müßte.

Wenn nur die effektiven Flächen der Wattetupfer insbesondere durch Heißluft zwecks Zusammenschluß erwärmt werden, wird entsprechend der geringeren Materialfläche weniger Energie benötigt.

Dieses Verfahren hat auch den Vorteil, daß eine günstige Randausbildung unter Vermeidung eines geschlossenen Randes erreichbar ist, weil erst nach der Ausstanzung oder Prägung der Umrißform die drucklose Aufschmelzung des Wattetupfers einschließlich des Randes erfolgt und dadurch auch der Rand nicht ausflusen kann. Bei der Ausstanzung bleibt der Rand des Wattetupfers schon wegen der Anteile von wenigstens an ihrer Oberfläche schmelzbaren Fasern offen.

Die Anlage zeichnet sich zur Durchführung des Verfahrens erfindungsgemäß dadurch aus, daß in der Verarbeitungsstrecke die Stanzvorrichtung vor der Erwärmungsvorrichtung angeordnet ist und daß die Transportstrecke der Abführungseinrichtung für in der Stanzvorrichtung anfallende Abfallmaterialien vorgesehen ist. Durch diese Anordnung werden die oben geschilderten Vorteile erreicht.

In weiterer Ausbildung wird für die Anlage gemäß obigen Darlegungen eine Auflockerungsvorrichtung zur Auflösung des Abfallmaterials in die Faserteile vorgesehen.

Hierbei handelt es sich um die Abfallmaterialien, die aus der Stanzvorrichtung kommen. Diese Auflockerungsvorrichtung ist im Rahmen der erfindungsgemäßen Anlage möglich, weil gemäß dem beschriebenen Verfahren auch thermoplastische Faserteile noch im originären Zustand, d.h. unangeschmolzen und unverschmolzen vorhanden sind.

In der bevorzugten Ausführungsform besitzt die Anlage als Erwärmungseinrichtung einen insbesondere trommelförmigen Siebträger und wenigstens ein Heißluftgebläse, wobei vorteilhaft in dem Siebträger Formausprägungen entsprechend der Umrißform der ausgestanzten Wattetupfer angeordnet sind. Dabei ist nur die Formausprägung als Sieb ausgebildet, während die erhabenen Teile eine geschlossene Abdeckung bilden. Das bedeutet, daß der Siebträger nur in dem Bereich durchlässig ist, in dem die Wattetupfer aufliegen.

Die Formausprägungen nehmen die Stanzstücke auf und halten sie in ihrer Form. Eine weitere vorteilhafte Ausführung besteht darin, den trommelförmigen Siebträger mit einer Schablone zu unterlegen, die entsprechend der Umrißform der ausgestanzten Wattetupfer Ausstanzungen aufweist, so daß die Heißluft nur durch diese Öffnungen und die darauf plazierten Wattetupfer strömen kann.

Der Antrieb des Siebträgers erfolgt vorteilhaft über ein Schrittgetriebe, das entsprechend der Hubgeschwindigkeit der Stanze gesteuert ist.

In der beschriebenen Ausführung ist in einer vorteilhaften Ausgestaltung die Stanzvorrichtung über der Erwärmungsvorrichtung und für beide Vorrichtungen ein synchronisierter Antrieb mit einem Schrittgetriebe für die die Erwärmungsvorrichtung bildende Siebtrommel angeordnet.

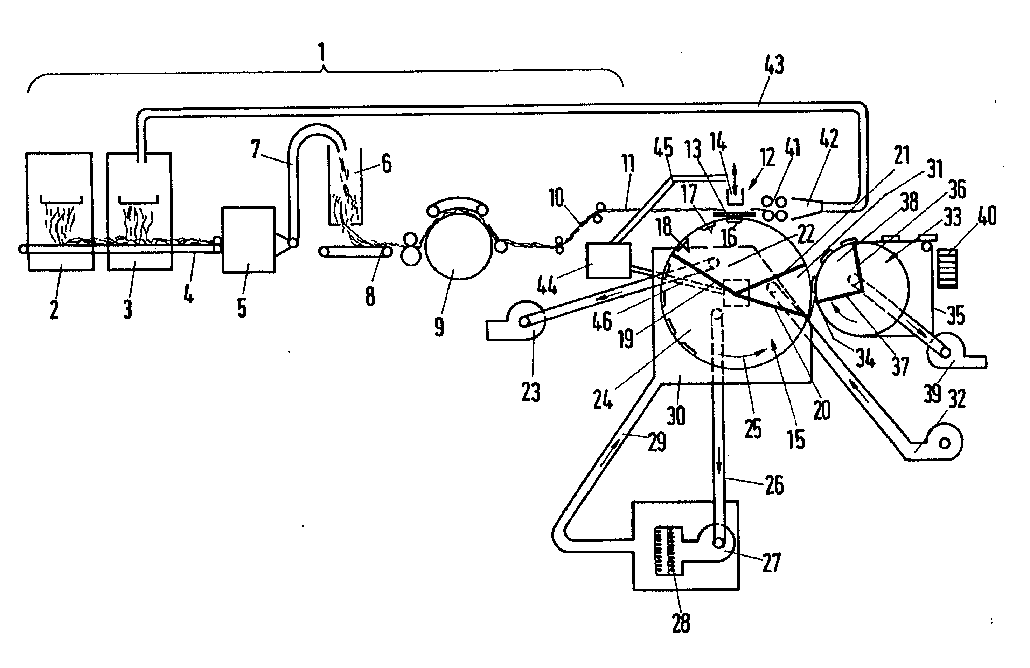

Ein Ausführungsbeispiel für die Anlage ist schematisch in der beigefügten Zeichnung dargestellt.

In dieser Zeichnung enthält die dargestellte Anlage, beginnend bei einer Faseraufbereitung bis zur Stapelung der Wattetupfer, als Teil eine Fertigungsstrecke 1 zur Herstellung eines Wattebandes. Diese Fertigungsstrecke enthält Wiegekastenspeiser 2, 3 an einem Förderband 4. In beiden Wiegekastenspeisern werden unter Bemessung verschiedene Fasern zugeführt, und zwar in einem 3 beispielsweise Baumwolle und in dem anderen 2 ein Anteil an wenigstens an ihrer Oberfläche schmelzbaren Fasern. Diese Transporteinrichtung 4 führt in ein Auflösungs- und Vermischungsorgan 5. Aus diesem wird das Material in eine Karden- bzw. Krempelbeschickung 6 durch ein Gebläserohr 7 überführt und durch Transportmittel 8 der eigentlichen Karde bzw. der Krempel 9 zugeführt. In üblicher Weise gelangt der damit gebildete Watteflor in eine Dubliervorrichtung, wie zum Beispiel Bandleger 10. Die so weit beschriebenen Teile 2 bis 10 bilden eine an sich bekannte Fertigungsstrecke zur Herstellung eines Wattebandes.

Das aus dem Bandleger 10 kommende Watteband 11 wird einer Stanzvorrichtung 12 mit einer Matrize 13 und einem Stempel 14 zugeführt, die in der beschriebenen Ausführung über dem Scheitel einer Siebtrommel 15 angeordnet sind. Diese Siebtrommel hat nach innen gerichtete Formausprägungen 16, 17, 18 um den Umfang verteilt. Sie ist nur im Bereich dieser Ausprägungen durchlässig, während die erhabenen Teile entweder durch eine Schablone abgedeckt oder luftundurchlässig ausgeführt sind. Diese Siebtrommel ist zugleich die Erwärmungsvorrichtung. Sie ist durch innerhalb ihres Mantels stationär angeordnete radiale Wände 19, 20, 21 in verschiedene Sektoren unterteilt.

Der Sektor 22 unterhalb der Stanzvorrichtung steht mit einem Saugventilator 23 in Verbindung, so daß die anfallenden Wattetupfer in den Formausprägungen gehalten werden, wenn sich die Siebtrommel entsprechend dem eingezeichneten Pfeil 25 dreht. Dabei gelangen die Wattetupfer in den Formausprägungen in den Bereich des Sektors 24. Dieser ist an den Saugstutzen 26 eines Saugventilators 27 angeschlossen, dem eine Erwärmungsvorrichtung 28, beispielsweise aus elektrischen Heizelementen nachgeschaltet ist. Die auf die Größenordnung von etwa 140°C erwärmte Luft wird durch eine Rohrverbindung 29 in einen die Siebtrommel 15 wenigstens teilweise im Bereich des Sektors 24 umgebenden Wärmekasten 30 geblasen, so daß die in Richtung der eingezeichneten Pfeile im Bereich der Formausprägungen in die Siebtrommel gelangende Luft nicht nur die Wattetupfer in den Formausprägungen hält, sondern die schmelzbaren Fasern auch drucklos aufschmilzt. Die Siebtrommel 15 wird über ein nicht dargestelltes Schrittgetriebe angetrieben, dessen Schrittschaltung mit der Auf- und Abwärtsbewegung des Stempels 14 synchronisiert ist, so daß jeweils bei einem Stanzvorgang eine Formausprägung unterhalb der Matrize angeordnet ist. Die resultierende Drehgeschwindigkeit der Siebtrommel 15 ist so gewählt, daß am Ende des Sektors 24 die Thermoverfestigung der Wattetupfer vollzogen ist.

Der Antrieb für diese Baugruppe ist schematisch mit 44 bezeichnet und steht über Funktionsverbindungen 45, 46 mit den Einzelantrieben der Stanzvorrichtung 12 und der Siebtrommel 15 in Verbindung.

Beim Weiterdrehen der Siebtrommel gelangen die Formausprägungen in den Sektor 31. Dieser Sektor steht mit einem Ventilator 32 in Verbindung, der kalte Luft einbläst, damit die noch in den Formausprägungen befindlichen Wattetupfer gekühlt und in einer Zone 34 des Zusammenlaufs mit einer anderen Siebtrommel 33 die Wattetupfer auf diese Trommel übertragen werden. Vor der Zone 34 kann der äußere Umfang der Siebtrommel 15 durch eine Verkleidung abgedeckt sein, um eine vorzeitige Ausgabe der Wattetupfer zu verhindern. Die Siebtrommel 33 ist von einem Siebband 35 umschlossen und durch Zwischenwände 36, 37 mit einem Sektor 38 versehen, der sich von der Zone 34 bis zu etwa dem oberen Scheitel der Siebtrommel 33 erstreckt. Dieser Sektor 38 steht mit einem Sauggebläse 39 in Verbindung, so daß in dem Sektor 38 Unterdruck herrscht, der die Wattetupfer auf dem Siebband 35 hält. Dieses Siebband führt die fertiggestellten Wattetupfer zu einer Ablage 40.

Hinter der Stanzvorrichtung 12 befindet sich für das Abfallmaterial, das noch bandförmig zusammengeschlossen ist, eine mit Abzugswalzen versehene Auflockerungsvorrichtung 41. In dieser wird das Abfallmaterial bzw. die Abfallmaterialbahn in ihre Faserbestandteile zerlegt. Von der Auflockerungsvorrichtung werden die aus dem Abfallmaterial gebildeten Faserstoffe über eine Fangvorrichtung 42 mit einem nicht dargestellten Gebläse durch eine Rohrleitung als Transportstrecke 43 dem Wiegekastenspeiser 3 zugeführt, wobei in der Wiegeeinrichtung die rückgeführte Menge gemessen und damit zugleich eine entsprechende Dosierung für eine neue Faserzuführung gesteuert wird.

Die dargestellte Anlage zeigt eine vorteilhafte Ausführungsform. Abwandlungen sind möglich. Zum Beispiel können aus einem Watteband mehrere Wattetupfer nebeneinander ausgestanzt werden. Statt des Stanzens ist der Einsatz eines Rotations-Schneidwerkzeuges möglich.

## Patentansprüche

1. Verfahren zur Herstellung von Gegenständen mit Saugfähigkeit unter Verwendung von Fasermaterial, wie Baumwolle, Viskose, mit Anteilen von wenigstens an ihrer Oberfläche schmelzbaren Fasern, bei dem Formstücke entsprechend dem Gegenstand aus einem Fasermaterialband aus dem Fasermaterial ausgestanzt oder geschnitten werden und ein Verschnitt als Abfall anfällt, ferner diese Abfallmaterialien in eine Fertigungsstrecke zur Herstellung eines Fasermaterialbandes zurückgeführt werden und im Verlauf dieses Verfahrens Wärme aufgebracht wird, durch welche die wenigstens an ihrer Oberfläche schmelzbaren Fasern verbunden werden, dadurch gekennzeichnet, daß als Gegenstände mit Saugfähigkeit Wattetupfer aus einem Watteband aus Fasermaterial mit Anteilen von wenigstens an ihrer Oberfläche schmelzbaren Fasern ausgestanzt und erst danach die bereits ausgestanzten Wattebandabschnitte, die jeweils einen Tupfer bilden, einer die Tupfer verfestigenden Wärmeaufbringung ausgesetzt werden, und daß das Abfallmaterial nach der Ausstanzung und vor der Wärmeaufbringung zurückgeführt wird.

2. Verfahren nach Anspruch 1, dadurch gekennzeichnet, daß die Tupfer mit kreisförmigem Umfang ausgestanzt werden.

3. Verfahren nach Anspruch 1, dadurch gekennzeichnet, daß Tupfer mit mehreckigen oder anderen Umrißformen mit gekrümmten Randlinien ausgestanzt werden.

4. Verfahren nach einem der Ansprüche 1 bis 3, dadurch gekennzeichnet, daß die Abfallmaterialien vor der Einführung in die Fertigungsstrecke nur aufgelockert werden.

5. Anlage zur Durchführung des Verfahrens nach einem der Ansprüche 1 bis 4 mit einer Fertigungsstrecke für die Herstellung eines Wattebandes mit einer Erwärmungsvorrichtung zum Zusammenschluß des Wattebandes durch Schmelz- und Verklebungsverbindungen zwischen Fasern an Kreuzpunkten und einer Stanzvorrichtung zum Ausstanzen von Wattetupfern sowie einer Transportstrecke zur Rückführrung von Abfallmaterialien in der Fertigungsstrecke, dadurch gekennzeichnet, daß in der Verarbeitungsstrecke die Stanzvorrichtung (12) vor der Erwärmungsvorrichtung (15) angeordnet ist und daß die Transportstrecke (43) der Abführungseinrichtung (42) für in der Stanzvorrichtung (12) anfallende Abfallmaterialien vorgesehen ist.

6. Anlage nach Anspruch 5, dadurch gekennzeichnet, daß in der Transportstrecke (43) eine Auflockerungsvorrichtung (41) zur Auflösung des Abfallmaterials in die Faserteile angeordnet ist.

7. Anlage nach Anspruch 5 oder 6, dadurch gekennzeichnet, daß als Erwärmungsvorrichtung (15) ein insbesondere trommelförmiger Siebträger und wenigstens ein Heißluftgebläse (27, 28) vorgesehen sind.

8. Anlage nach Anspruch 7, dadurch gekennzeichnet, daß in dem Siebträger (15) Formausprägungen (16 bis 18) entsprechend der Umrißform der ausgestanzten Wattetupfer angeordnet sind.

9. Anlage nach Anspruch 7 oder 8, dadurch gekennzeichnet, daß der Siebträger (15) nur in dem Bereich durchlässig ist, in dem die Wattetupfer aufliegen.

10. Anlage nach einem der Ansprüche 5 bis 7, dadurch gekennzeichnet, daß die Stanzvorrichtung (12) über der Erwärmungsvorrichtung (15) und für beide Vorrichtungen ein synchronisierter Antrieb mit einem Schrittgetriebe für die Siebtrommel angeordnet ist.

## Claims

1. A method of making absorbent articles by using fibrous material, i.e, cotton, viscose including a share of fibres which have at least a melting surface, in which method preforms corresponding to the article are stamped or cut out of a web of fibrous material leaving waste cuttings to be recycled to a manufacturing station making said fibrous material web, and subjecting said preforms to heat for joining said fibres which have at least a melting surface, characterized in that cotton wool pads are cut out of a cotton wool web of fibrous material including a share of fibres which have at least a melting surface, said pads defining said absorbent articles, that further the pieces each forming said pads are subsequently subjected to heat for solidifying said pads and that the waste material is recycled after the cutting step and before the heating step.

2. The method of claim 1, characterized in that circular pads are cut.

3. The method of claim 1, characterized in that rectangular pads or, respectively pads having different peripheral profiles comprising curved edges are cut.

4. The method of one of claims 1 to 3, characterized in that the waste material is merely loosened up before returning to the manufacturing station.

5. An apparatus for performing the method of one of claims 1 to 4 comprising a manufacturing station for making a cotton wool web, a heating means for solidifying the web by melting and adhering joining said fibres at their crossing points and a cutting means for cutting cotton wool pads, and further a transfer means for recycling waste material to the manufacturing station, characterized in that said cutting means (12) is located upstream of the heating means (15) in said manufacturing station and that the transfer means (43) of the recycling means (42) is provided to transfer the waste material produced in the cutting means (12).

6. The apparatus of claim 5, characterized in that a loosening means (41) to disagregate the waste material to fibres is provided at said transfer means (43).

7. The apparatus of claim 5 or 6, characterized in that said heating means (15) comprises a sieve, in particular a drum-shaped sieve and at least a hot air blower (27, 28).

8. The apparatus of claim 7, characterized in that said sieve (15) comprises mould stampings (16 to 18) corresponding to the circumference of the cotton wool pads cut out.

9. The apparatus of claim 7 or 8, characterized in that said sieve (15) is perforated in areas supporting said pads.

10. The apparatus of one of claims 5 to 7, characterized in that said cutting means (12) is provided above said heating means (15) and that a synchronised drive means including a step gear for the sieve drum is provided for both said means.

## Revendications

1. Procédé de production d'articles absorbants par utilisation de matière fibreuse telle que coton, viscose, une partie des fibres étant fusibles au moins à leur surface, selon lequel on découpe par matriçage ou avec un outil coupant, à partir d'une bande de la matière fibreuse, des pièces de forme correspondant à l'article à produire, avec production d'une chute, on recycle ensuite la matière de ces chutes dans un banc de production pour obtenir une bande de matière fibreuse, et, pendant le cours de ce processus, on fait un apport de chaleur grâce auquel on lie les fibres fusibles au moins à leur surface, caractérisé en ce qu'on découpe par matriçage, comme articles absorbants, des tampons d'ouate, à partir d'une bande d'ouate de matière fibreuse avec une partie de fibres fusibles au moins à leur surface, et immédiatement après, on soumet les pièces découpées à partir de la bande d'ouate, et dont chacune constitue un tampon, à un apport de chaleur qui consolide le tampon, et en ce que la matière des chutes est envoyée au recyclage après le découpage et avant l'apport de chaleur.

2. Procédé selon la revendication 1, caractérisé en ce que les tampons sont découpés avec un contour circulaire.

3. Procédé selon la revendication 1, caractérisé en ce que les tampons sont découpés avec une forme polygonale ou autre, à contours arrondis.

4. Procédé selon l'une des revendications 1 à 3, caractérisé en ce que les chutes sont seulement désagrégées avant d'être introduites dans le banc de production.

5. Installation pour la mise en oeuvre du procédé selon l'une des revendications 1 à 4, comprenant un banc de production pour l'obtention d'une bande d'ouate, un dispositif de chauffage pour rassembler la bande d'ouate en reliant les fibres à leurs points de contact par fusion et collage, et un dispositif de découpe par matriçage, pour découper des tampons d'ouate, ainsi qu'un moyen de transport pour le recyclage des chutes vers le banc de production, caractérisée en ce que, sur la ligne de travail, le dispositif de découpe par matriçage (12) est placé avant le dispositif de chauffage (15), et en ce que dans le moyen de transport (43) il est prévu un dispositif (42) d'évacuation pour les chutes formées dans le dispositif de découpe par matriçage (12).

6. Installation selon la revendication 5, caractérisée en ce qu'un dispositif de désagrégation (41) pour réduire les chutes en leurs composants fibreux est placé dans le moyen de transport (43).

7. Installation selon la revendication 5 ou 6, caractérisée en ce qu'il est prévu, comme dispositif de chauffage (15) un porte-tamis, en particulier en forme de tambour, et au moins un moyen de soufflage d'air chaud.

8. Installation selon la revendication 7, caractérisée en ce que des empreintes (16 à 18) de forme correspondant au contour des tampons d'ouate découpés par matriçage sont disposées sur le porte-tamis (15).

9. Installation selon la revendication 7 ou 8, caractérisée en ce que le porte-tamis (15) n'est perméable que dans la zone où se trouvent les tampons d'ouate.

10. Installation selon l'une des revendications 5 à 7, caractérisée en ce que le dispositif de découpage par matriçage est placé au-dessus du dispositif de chauffage (15), et en ce qu'un entraînement synchronisé est prévu pour ces deux dispositifs, avec un entraînement pas-à-pas pour le tambour porte-tamis.

EP 0 310 923 B1